# EUROPÄISCHE PATENTSCHRIFT

(11) **EP 0 496 705 B1**
(45) Veröffentlichungstag und Bekanntmachung des Hinweises auf die Patenterteilung: **13.09.1995**
(21) Anmeldenummer: 92810041.1
(22) Anmeldetag: 23.01.1992
(51) Int. Cl.: A61K 9/48, A61K 35/78

(54) **Masse, welche insbesondere für die Einkapselung in ein festes Umhüllungsmaterial geeignet ist, und Verfahren zur Herstellung dieser Masse**
Composition especially adapted for encapsulation in a solid shell and process for preparing same
Composition spécialement apte pour être encapsulée dans une enveloppe solide et son procédé de préparation

(30) Priorität: 24.01.1991 CH 208/91
(43) Veröffentlichungstag der Anmeldung: 29.07.1992
(73) Patentinhaber: Emil Flachsmann AG, CH-8820 Wädenswil (CH)
(72) Erfinder: Honerlagen, Hans Josef, CH-6314 Unterägeri (CH); Bolardt, früher Skrceny Miroslav, CH-8800 Thalwil (CH)
(74) Vertreter: Zink-Wild, Markus Peter

(56) Entgegenhaltungen:
- EP-A- 0 086 468
- EP-A- 0 332 478
- EP-A- 0 343 575
- EP-A- 0 347 493
- EP-A- 0 370 284
- EP-A- 0 464 274
- DE-A- 2 337 617
- DE-A- 2 548 820
- DE-A- 2 614 864
- US-A- 3 202 578

## Beschreibung

Die vorliegende Erfindung betrifft eine Masse, welche insbesondere für die Einkapselung in ein festes Umhüllungsmaterial geeignet ist, und ein Verfahren zur Herstellung dieser Masse.

Trockene Substanzen - hierunter fallen auch Pflanzenextrakte- sollten zur Einarbeitung in Weichgelatinekapseln im Prinzip eine Korngrösse von maximal 100 mµ haben. Maximal 20 Gew.-% der Körner dürfen eine Korngrösse von maximal 180 mµ haben. Grössere Körner verstopfen die feinen Düsen und verursachen Betriebsunterbrüche.

Zur Vermahlung auf die jeweils gewüschte Korngrösse werden in der Industrie normalerweise Doppelstiftmühlen verwendet. Diese Mühlen - wie Mühlen allgemein-benötigen grosse Mengen an Arbeitsluft. Dabei dient das zu vermahlende, sich in der Mühle befindliche Gut als Filter, wodurch dieses Gut zwangsläufig kontaminiert wird. Ausgenommen davon sind die Arbeitsweisen unter den sehr teuren Reinraumbedingungen. Die genannte Kontamination umfasst auch die mikrobiologische Verunreinigung.

Extrakte aus Pflanzen mussten bis anhin zur Einarbeitung in Kapseln vollständig getrocknet und, wie oben erwähnt, fein gemahlen werden. Die Trocknungs- und die Mahlkosten sind enorm. Zudem treten beim Trocknen Wirkstoffverluste und beim Mahlen unweigerlich Massen/Mengenverluste an gewünschtem Material auf. In Bezug auf die Mikrobiologie kann keine verbindliche Qualitätssicherung gegeben werden.

Ferner ist bekannt, dass Substanzen oder Substanzgemische, welche noch eine Restlösungsmittelmenge enthalten, wie beispielsweise Wasser, einen niederen Alkohol, Aceton, Methylethylketon, etc., wegen der Klumpenbildung nicht vermahlen werden können, und somit auch nicht in dieser Form eingekapselt werden können.

Es ist ein Ziel der vorliegenden Erfindung, die oben genannten Nachteile zu überwinden.

Es soll eine Masse, welche insbesondere für die Einkapselung in ein festes Umhüllungsmaterial, beispielsweise Gelatine, wie etwa Weichgelatine, geeignet ist, zur Verfügung gestellt werden.

Diese Masse soll ohne die oben erwähnten Trocknungs- und Mahlschritte des die aktiven Verbindungen enthaltenden Extraktes erhalten werden. Es soll also kein Trockenextrakt eingekapselt werden.

Ebenso soll ein einfaches und billiges Verfahren zur Herstellung dieser Masse zur Verfügung gestellt werden.

Die erfindungsgemässe Masse - auch Gemisch genannt -, welche insbesondere für die Einkapselung in ein festes Umhüllungsmaterial geeignet ist, ist dadurch gekennzeichnet, dass sie
- wenigstens einen weder getrockneten noch gemahlenen Teil- oder Vollextrakt aus frischen und/oder getrockneten Pflanzen oder Teilen davon, und
- wenigstens ein Trägermaterial für den genannten Teil- oder Vollextrakt enthält,
   wobei das genannte Trägermaterial verkapselbar und gegenüber dem genannten Extrakt inert sein muss, und ausgewählt ist aus der Gruppe, bestehend aus
   - Polyethylenglycolen der allgemeinen Formel

      H-[-O-CH₂-CH₂-]ₙ-O-H

      worin n eine ganze Zahl im Bereich von 100 bis 3000, insbesondere von 100 bis 400, bedeutet,
      einschliesslich Gemische davon,
   - Fettsäureestern von Polyglycerinen, einschliesslich Gemische davon,
   - Siliconölen, einschliesslich Gemische davon,
   - Lecithinen, einschliesslich Gemische davon,
   - Sorbitanfettsäureestern, auch
      Sorbate von Fettsäuren genannt,
   - Polysorbaten von Fettsäuren,
   - Wachse,
   - Polyglycerin,
   - Triglyceride,
   - Fettsäuren und
   - fette Oele.

Bevorzugte Ausführungsformen dieser Masse sind in den abhängigen Ansprüchen definiert.

Mit dieser erfindungsgemässen Masse kann ein nicht vollständig getrockneter Pflanzenextrakt eingekapselt werden. Damit werden einerseits Trocknungs- und Mahlkosten eingespart, und andererseits werden durch den Wegfall des Mahlens eine Qualitätssicherung und eine Qualitätsverbesserung in Bezug auf die mikrobiologischen Verunreinigungen erzielt. Ferner werden die Materialverluste beim Trocknen und beim Mahlen vermieden. Mit der erfindungsgemässen Masse ist es erstmals möglich geworden, Restlösungsmittelmengen enthaltende Teil- oder Vollextrakte aus frischen und/oder getrockneten Pflanzen oder Teilen davon zu verkapseln.

Das Trägermaterial für den Teil- oder Vollextrakt muss verkapselbar und gegenüber dem genannten Extrakt inert sein. So kann ein mit dem Extraktionsmittel Propylenglycol erhaltener Extrakt nicht verkapselt werden, weil solche Kapseln deformiert werden.

Als Trägermaterial für den genannten Teil-oder Vollextrakt werden die Polyethylenglycole, wie etwa Polyethylenglycol 400, bevorzugt.

Mit diesem Trägermaterial wird keine Deformation der Kapseln beobachtet.

Bezüglich den Polyethylenglycolen, welche auch Macrogola genannt werden, sei auf den Artikel in "Phamacopoea Helvetica" 7. Auflage vom 1. Januar 1990 verwiesen.

Das erfindungsgemässe Verfahren zur Herstellung der erfindungsgemässen Masse ist dadurch gekennzeichnet, dass dass man
- in einem ersten Schritt einen auf übliche Art erhaltenen, wenigstens ein Lösungsmittel enthaltenden Teil- oder Vollextrakt aus frischen und/oder getrockneten Pflanzen oder Teilen davon teilweise einengt,
- in einem zweiten Schritt den so erhaltenen Rückstand mit wenigstens einem Trägermaterial für den genannten Teil- oder Vollextrakt vermischt, wobei das genannte Trägermaterial verkapselbar und gegenüber dem genannten Extrakt inert sein muss, und
- in einem dritten Schritt das so erhaltene Gemisch nahezu vollständig vom Extraktionsmittel oder Extraktionsmittelgemisch befreit.

Bevorzugte Ausführungsformen dieses Verfahrens sind in den abhängigen Ansprüchen definiert.

Das vollständige Einengen auf praktische Lösungsmittelfreiheit erfolgt also erst nach der Zugabe des Trägermaterials.

Es ist auch möglich, einen Teil- oder Vollextrakt in konzentrierter Form zu lagern, ihn dann mit dem gewünschten lösungsmittel oder dem gewünschten Lösungsmittelgemisch in gewüschtem Mass zu verdünnen und ihn dann im erfindungsgemässen Verfahren zu verwenden.

Ein wenigstens teilweiser eingeengter Teil-oder Vollextrakt kann auch bequem zum Ort seiner Verarbeitung transportiert werden, und dann hier mit der Trägermaterial-Komponente vermischt und danach nahezu vollständig eingeengt werden.

Solche Ausführungsformen werden auch als unter den Schutzbereich dieser Erfindung fallend betrachtet.

Für den Fall, dass mit der Trägermaterial-Komponente selbst, beispielsweise ein Polyethylenglycol, wie etwa Polyethylenglycol 400, die gewünschten aktiven Verbindungen aus den Pflanzen herausgelöst werden können, ist es möglich, die Pflanzen direkt mit der Trägermaterial-Komponente zu extrahieren. Gegebenenfalls kann auch ein Gemisch an Polyethylenglycol mit einem niedermolekularen Lösungsmittel, z.B. Ethanol, H₂O, verwendet werden. Danach müssen dann lediglich noch die Festkörper abgetrennt werden, beispielsweise mittels Zentrifugieren oder Filtrieren, und die gewünschte Konzentration an aktiven Verbindungen eingestellt werden.

Die Einarbeitung der erfindungsgemässen Masse als solche, oder gegebenenfalls vermischt mit einem Zusatz-und/oder Hilfsstoff, in übliche Umhüllungsmaterialien, wie etwa Weichgelatine, für Kapseln kann gemäss den im Stand der Technik bekannten Methoden erfolgen; siehe beispielsweise DE PS 38 18 022.

Es ist bevorzugt, Teil- oder Vollextrakte zu verwenden, wie sie in der Europäischen Patentanmeldung Nr. 88 118 469.1-2107, Veröffentlichungs-Nr. 0 347 493 beschrieben sind. Mit der hierin beschriebenen Lehre ist es u.a. möglich, einen Knoblauchextrakt (Bulbus Alii sativi) herzustellen, welcher das Alliin in unveränderter Form enthält, d.h., die Reaktion zwischen dem Alliin und dem Enzym Alliinase zum Produkt Allicin hat noch nicht stattgefunden. Ein solcher Knoblauchextrakt kann nun gemäss der vorliegenden Erfindung eingekapselt werden.

Die nachfolgenden Beispiele sollen die vorliegende Erfindung illustrieren.

### Beispiel 1

30 kg frische Knoblauchzehen (Bulbus Allii sativ.) wurden in 150 kg Methanol gegeben. Die Zerkleinerung der Knoblauchzehen erfolgte unter Methanol mittels einer Schneidvorrichtung. Danach wurde noch eine Stunde unter Rühren bei Raumtemperatur extrahiert, dann filtriert.

Das Filtrat wurde unter vermindertem Druck unterhalb 60°C auf 1/5 des eingesetzten Drogengewichtes eingeengt = 6 kg. Diesem aufkonzentrierten Menstruum wurden 1,0 kg Polyethylenglycol 400 und 3,5 kg Decaglycerinmonoisostearat zugemischt und diese Mischung wurde unter vermindertem Druck unterhalb 60°C auf praktische Wasser- und Methanolfreiheit eingeengt.

Erhalten wurden 6,8 kg einer Masse, welche sich zur direkten Einarbeitung in Weichgelatinekapseln eignet.

Die analytischen Daten sind wie folgt:

| | |
|---|---|
| Alliin | 1,9 % m/m |
| Restwasser | 0,9 % m/m |
| Restmethanol | 0,04 % m/m. |

### Beispiel 2

30 kg frische Knoblauchzehen (Bulbus Allii sativ.) wurden in 150 kg Methanol gegeben. Die Zerkleinerung der Knoblauchzehen erfolgte unter Methanol mittels einer Schneidvorrichtung. Danach wurde noch eine Stunde unter Rühren bei Raumtemperatur extrahiert, dann filtriert.

Das Filtrat wurde unter vermindertem Druck unterhalb 60°C auf 5 kg eingeengt. Diesem Konzentrat wurden 5,4 kg Polyglycerin zugesetzt. Nach Homogenisierung und zweistündiger Stehzeit bei Raumtemperatur wurde von entstandenen Ausfällungen mittels Zentrifugation separiert. Das Zentrifugat wurde unter vermindertem Druck bei max. 60°C auf praktische Wasser- und Methanolfreiheit eingeengt.

Erhalten wurden 7,1 kg einer klaren, braungefärbten Masse, welche sich zur direkten Einarbeitung in Weichgelatinekapseln eignet.

Die analytischen Daten sind wie folgt:

| | |
|---|---|
| Alliin | 1,8 % m/m |
| Restwasser | 0,8 % m/m |
| Restmethanol | 0,04 % m/m. |

### Beispiel 3

5,5 kg Flores Chamomillae (Matricaria chamomilla) wurden mit 99,8 %-igem Ethanol nach dem Perkolationsverfahren extrahiert. Das Perkolat wurde über Molekularsieb (4 Angström) entwässert und dann unter Vakuum bei max. 40°C konzentriert.

Erhalten wurden 0,411 kg Konzentrat. Dem Konzentrat wurden 0,462 kg Polyethylenglycol 400 zugesetzt.

Erhalten wurde 0,785 kg einer ersten, viskosen, lipophilen Masse mit einem Restethanolgehalt von 0,5 %.

Die so extrahierten Kamillenblüten wurden anschliessend mit einem 1:1 m/m Gemisch von Wasser und Ethanol zur Gewinnung der polaren Anteile nach dem Verfahren der Perkolation extrahiert. Das Perkolat wurde unter Vakuum bei maximal 70°C auf einen Trockensubstanzgehalt von 60 % eingeengt.

Erhalten wurden 0,139 kg Konzentrat.

Diesem Konzentrat wurden 0,597 kg Polyethylenglycol 400 zugesetzt, homogenisiert und anschliessend unter Vakuum bei max. 70°C bis zur praktischen Wasserfreiheit behandelt.

Erhalten wurden 1,450 kg einer zweiten, viskosen, polaren Masse mit einem Wassergehalt von 2,1 %.

Zur Einkapselung in Weichgelatinekapseln wurden die beiden oben genannten Massen vereinigt und homogenisiert. Die analytischen Daten dazu sind wie folgt:

| | |
|---|---|
| Apigenin | 0,15 % m/m |
| Apigenin-7-glucosid | 0,55 % m/m |
| (-)-α-Bisabolol | 0,037 % m/m |
| ätherisches Oel | 1,0 % V/m |
| Chamazulen | 0,0079 % m/m |
| Restwasser | 1,36 % m/m |
| Restethanol | 0,17 % m/m. |

### Beispiel 4

10 kg gemahlene Weidenrinde (Cortex salicis) wurden mit heissem Wasser von 90°C nach dem Perkolationsverfahren extrahiert. Das Perkolat wurde auf 1/3 des eingesetzten Drogengewichtes unter vermindertem Druck bei max. 60°C eingeengt = 3,3 kg. Diesem Konzentrat wurden 0,2 kg Polyethylenglycol 400 zugesetzt und homogenisiert.

Es traten leichte Ausfällungen auf, welche abzentrifugiert wurden. Das Zentrifugat wurde dann unter vermindertem Druck bei max. 60°C weiterkonzentriert.

Erhalten wurden 0,580 kg einer klaren viskosen Masse, welche sich zur direkten Einarbeitung in Weichgelatinekapseln eignet.

Die analytischen Daten sind wie folgt:

| | |
|---|---|
| Restwassergehalt | 3,3 % m/m |
| Salicingehalt | 11,8 % m/m. |

### Beispiel 5

10 kg gemahlene Weidenrinde (Cortex salicis) wurden mit heissem Wasser von 90°C nach dem Perkolationsverfahren extrahiert. Das Perkolat wurde auf 1/10 des eingesetzten Drogengewichtes unter vermindertem Druck bei max. 60°C eingeengt = 1,0 kg. Diesem Konzentrat wurden 0,2 kg Polyethylenglycol 400 zugesetzt und homogenisiert.

Es traten leichte Ausfällungen auf, welche abzentrifugiert wurden. Das Zentrifugat wurde dann unter vermindertem Druck bei max. 60°C weiterkonzentriert.

Erhalten wurden 0,57 kg einer klaren viskosen Masse, welche sich zur direkten Einarbeitung in Weichgelatinekapseln eignet.

Die analytischen Daten sind wie folgt:

| | |
|---|---|
| Restwassergehalt | 3,4 % m/m |
| Salicingehalt | 11,3 % m/m. |

### Beispiel 6

1 kg Isländisches Moos (Lichen islandicus) (gemahlen) wurde nach dem Verfahren der Perkolation bei Raumtemperatur mit 9 kg einer Mischung, bestehend aus 7,2 kg Wasser und 1,8 kg Polyethylenglycol 400, extrahiert. Das Perkolat wurde unter vermindertem Druck bei max. 60°C bis auf einen Wassergehalt von unter 5 % m/m eingeengt.

Erhalten wurden 1,58 kg eines klaren Extraktes mit einem Restwassergehalt von 3,5 % m/m. Dieser Extrakt kann zur direkten Einarbeitung in Weichgelatinekapseln verwendet werden.

### Beispiel 7

1 kg Fenchelfrüchte (Foeniculi vulg.) zerstossen, wurden mit 3 kg einer Mischung, bestehend aus 1,95 kg Polyethylenglycol 400 und 1,05 kg Ethanol mind. 98 %-ig m/m, nach dem Verfahren der Perkolation bei Raumtemperatur extrahiert. Das Perkolat wurde bei vermindertem Druck bei max. 60°C bis auf einen Ethanolgehalt von unter 0,5 % m/m eingeengt.

Erhalten wurden 1,6 kg eines klaren stark nach Fenchel schmeckenden Extraktes mit einem Restethanolgehalt von 0,4 % m/m. Dieser Extrakt kann zur direkten Einarbeitung in Weichgelatinekapseln verwendet werden.

### Beispiel 8

15 kg Muskatblüten (Flores Macidis) wurden mit 20 kg Ethanol mind. 99 %-ig durchfeuchtet und über Nacht stehen gelassen.

Am nächsten Morgen wurde die feuchte Droge in ein Perkolationsrohr gefüllt und dann mit 130 Liter Ethanol mind. 99 %-ig bei Raumtemperatur perkoliert. Das Perkolat wurde über 13 kg Molekularsieb (3 Angström) entwässert und anschliessend unter vermindertem Druck bei max. 40°C auf einen Ethanolgehalt von unter 0,5 % m/m eingeengt.

Erhalten wurden 4,86 kg Extrakt mit 24,6 % V/m ätherischem Oel.

Diese so hergestellten 4,86 kg Extrakt wurden mit 1,2 kg Sojaöl gemischt. Erhalten wurde eine homogene stabile Masse, welche sich zur direkten Einarbeitung in Weichgelatinekapseln eignet.

### Beispiel 9

15 kg Nelkenblüten (Flores Caryophylli) wurden mit 20 kg Ethanol mind. 99 %-ig durchfeuchtet und über Nacht stehen gelassen.

Am nächsten Morgen wurde die feuchte Droge in ein Perkolationsrohr gefüllt und dann mit 130 Liter Ethanol mind. 99 %-ig bei Raumtemperatur perkoliert. Das Perkolat wurde über 13 kg Molekularsieb (3 Angström) entwässert und anschliessend unter vermindertem Druck bei max. 40°C auf einen Ethanolgehalt von unter 0,5 % m/m eingeengt.

Erhalten wurden 2,35 kg Extrakt mit 70 % V/m ätherischem Oel. Diese so hergestellten 2,35 kg Extrakt wurden mit 1,0 kg Lecithin gemischt. Erhalten wurde eine homogene stabile Masse, welche sich zur direkten Einarbeitung in Weichgelatinekapseln eignet.

## Patentansprüche (Patentansprüche für folgende(n) Vertragsstaat(en): AT, BE, CH, LI, DE, DK, FR, GB, GR, IT, LU, MC, NL, PT, SE)

1. Masse, welche insbesondere für die Einkapselung in ein festes Umhüllungsmaterial geeignet ist, dadurch gekennzeichnet, dass sie
- wenigstens einen weder getrockneten noch gemahlenen Teil- oder Vollextrakt aus frischen und/oder getrockneten Pflanzen oder Teilen davon, und
- wenigstens ein Trägermaterial für den genannten Teil- oder Vollextrakt enthält,
wobei das genannte Trägermaterial verkapselbar und gegenüber dem genannten Extrakt inert sein muss, und ausgewählt ist aus der Gruppe, bestehend aus
- Polyethylenglycolen der allgemeinen Formel
H-[-O-CH₂-CH₂-]ₙ-O-H
worin n eine ganze Zahl im Bereich von 100 bis 3000, insbesondere von 100 bis 400, bedeutet,
einschliesslich Gemische davon,
- Fettsäureestern von Polyglycerinen, einschliesslich Gemische davon,
- Siliconölen, einschliesslich Gemische davon,
- Lecithinen, einschliesslich Gemische davon,
- Sorbitanfettsäureestern, auch
- Sorbate von Fettsäuren genannt,
- Polysorbaten von Fettsäuren,
- Wachse,
- Polyglycerin,
- Triglyceride,
- Fettsäuren und
- fette Oele.

2. Masse nach Anspruch 1, dadurch gekennzeichnet, dass sie noch Wasser enthält, insbesondere in einer Menge bis zu 10 Gew.-%, vorzugsweise in einer Menge bis zu 5 Gew.-%.

3. Masse nach einem der Ansprüche 1 bis 2, dadurch gekennzeichnet, dass sie noch wenigstens ein organisches Lösungsmittel enthält, insbesondere in einer solchen Menge, welche dem gesetzlich maximal zugelassenen Wert entspricht oder vorzugsweise diesen unterschreitet, wobei Mengen von maximal 0,5 Gew.-% Ethanol, und/oder maximal 0,05 Gew.-% Methanol, und/oder maximal 0,05 Gew.-% 2-Propanol, und/oder maximal 20 ppm Methylethylketon bevorzugt sind.

4. Masse nach einem der Ansprüche 1 bis 3, dadurch gekennzeichnet, dass das Umhüllungsmaterial Gelatine ist, beispielsweise Weichgelatine oder Hartgelatine, wobei Weichgelatine bevorzugt ist.

5. Masse nach einem der Ansprüche 1 bis 4, dadurch gekennzeichnet, dass die Pflanzen Heil- oder Gewürzpflanzen sind, und insbesondere ausgewählt sind aus der Gruppe bestehend aus
Abelmoschus moschatus L. (Semen)
Acorus calamus (Rhizom)
Aesculus hippocastanum L. (Semen)
Allium-Arten (z.B. A. cepa L., A. ursinum L., A. sativum L.:Bulbus)
Alpinia officinarum Hance (Rhizom)
Arctostaphylos uva-ursi Spreng. (Folium)
Arnica montana L. (Flos)
Artemisia absinthium L. (Herba)
Artemisia dracunculus L. (Herba)
Atropa belladonna L. (Folium)
Berberis vulgaris L. (Cortex, Radix)
Betula-Arten (Folium)
Brassica nigra (L.) Koch (Semen)
Carum carvi L. (Fructus)
Cetraria islandica (L.) Ach.
Chrysanthemum vulgare Asch. (Herba)
Cinnamomum-Arten (Cortex)
Citrus-Arten (Folium, Flavedo, Fruct.)
Copaifera reticulata Ducke (Balsam)
Coriandrum sativum L. (Fructus)
Cucurbita pepo L. (Semen)
Cuminum cyminum L. (Fructus)
Curcuma-Varietäten (Rhizoma)
Cusparia officinalis (Willd.) Eng. (Cortex)
Dipterocarpus tubinatus Gaertn. (Balsamum)
Drosera-Arten (D. rotundifolia L., D.ramentacea Burch; Herba)
Echinacea angustifolia D.C. (Radix)
Echinacea purpurea (L.) Moench (Radix)
Elettaria cardamonum (L.) White et Mathon (Fructus)
Equisetum arvense L. (Herba)
Eucalyptus globulus Labill. (Folium)
Fagopyrum vulgare Hill. (Herba)
Foeniculum vulgare Miller (Fructus)
Gaultheria procumbens L. (Folium)
Ginkgo biloba L. (Folium)
Hamamelis virginiana L. (Cortex, Folium)
Hedeoma pulegioides (L.) Pers. (Herba)
Herniaria glabra L. (Herba)
Humulus lupulus L. (Flos, Glandulae)
Hypericum perforatum L. (Herba)
Hysopus officinalis L. (Herba)
Ilex paraguariensis St. Hil. (Folium mate)
Illicium verum Hook. f. (Fructus)
Iluna helenium L. (Rhizoma)
Iris pallida Lam. (Rhizoma)
Jasminum grandiflorum L. (Flos)
Laurus nobilis L. (Folium, Fructus)
Lavendula officinalis, weitere Arten (Flos)
Lawsonia inermis L. (Folium)
Levisticum officinale Koch (Radix)
Maleleuca: div. Variet. (Folium)
Matricaria chamomilla L. (Flos)
Melilotus officinalis (L.) Lam. em. Thuill. (Herba)
Mentha-Arten und ihre Varietäten (Folium)
Myristica fragrans Houttuyn (Arillus, Semen)
Myrtus communis L. (Folium)
Ocimum basilicum L. (Herba)
Ocotea sassafras (Cortex)
Oenanthe aquatica (L.) Poir (Fructus)
Olibanum (Resinum)
Ononis spinosa L. (Radix)
Origanum-Varietäten (Herba)
Orthosiphon stamineus Benth. (Herba)
Panax ginseng Meyer (Radix)
Petroselinum crispum (Mill.) Nym. (Fructus, Herba)
Phaseolus vulgaris L. (Fructus sine Semine)
Pimenta dioica (L.) Merill (Fructus)
Pimpinella anisum L. (Semen)
Piper angustifolium Ruiz. et Pavon. (Folium)
Piper methysticum Forster (Radix)
Pogostemon patchouli Pell. (Folium)
Prunus laurocerasus L. (Folium)
Rhus aromatica Ait. (Cortex)
Rosmarinus officinalis L. und ihre Subspecies (Folium)
Rubia tinctorum L. (Radix)
Rubus fructicosus L. (Folium)
Ruta graveolens L. (Herba)
Sabal serulata Benth et Hook (Fructus)
Salix alba L. (Cortex) und alle Arten
Salvia-Varietäten (Folium)
Santalum album L. (Lignum)
Sarothamnus scoparius (L.) Wimmer (Herba)
Sassafras albidum (Nutt.) Nees (Lignum)
Satureja hortensis L. (Herba)
Scopolia carniolica Jacq. (Radix)
Solidago serotina Ait. (Herba)
Solidago virgaurea L. (Herba)
Syzygium aromaticum Merr. et Perry (Flores, Folium)
Taraxacum officinale Web. (Herba und Radix)
Thymus serpyllum L. (Herba)
Thymus vulgaris L. Herba
Tilia cordata Mill. und T. platyphyllos Scop. (Flos)
Urtica dioica L. (Folium, Radix)
Valeriana officinalis und ihre Varietäten (Radix)
Zingiberis officinale Roscoe (Rhizoma).

6. Masse nach einen der Ansprüche 1 bis 5, dadurch gekennzeichnet, dass das Gewichtsverhältnis von genannten Extrakt zu genannten Trägermaterial von 20 bis 200, insbesondere von 30 bis 150 , vorzugsweise von 40 bis 150, beträgt.

7. Masse nach einem der Ansprüche 1 bis 6, dadurch gekennzeichnet, dass das Trägermaterial ein Polyethylenglycol ist, vorzugsweise Polyethylenglycol mit einem mittleren Polymerisationsgrad von 100 bis 3000, insbesondere 400.

8. Masse nach einem der Ansprüche 1 bis 7, dadurch gekennzeichnet, dass sie noch wenigstens einen Zusatz- und/oder Hilfsstoff enthält, insbesondere ausgewählt aus der Gruppe, bestehend aus Emulgatoren, Stabilisatoren, Antioxidantien, Farbstoffen und Aromen.

9. Verfahren zur Herstellung einer Masse nach einem der Ansprüche 1 bis 8, dadurch gekennzeichnet, dass man
- in einem ersten Schritt einen auf übliche Art erhaltenen, wenigstens ein Extraktionsmittel enthaltenden Teil- oder Vollextrakt aus frischen und/oder getrockneten Pflanzen oder Teilen davon teilweise einengt,
- in einem zweiten Schritt den so erhaltenen Rückstand mit wenigstens einem Trägermaterial für den genannten Teil- oder Vollextrakt vermischt, wobei das genannte Trägermaterial verkapselbar und gegenüber dem genannten Extrakt inert sein muss, und
- in einem dritten Schritt das so erhaltene Gemisch nahezu vollständig vom Extraktionsmittel oder Extraktionsmittelgemisch befreit.

10. Verfahren nach Anspruch 9, dadurch gekennzeichnet, dass man am Ende des zweiten Schrittes einen gegebenenfalls entstandenen Niederschlag entfernt, beispielsweise mittels Filtration oder Zentrifugation.

11. Verfahren nach einem der Ansprüche 9 bis 10, dadurch gekennzeichnet, dass im ersten Schritt auf einen Trockensubstanzgehalt von etwa 20 bis 40 Gew.-% eingeengt wird.

12. Verfahren nach einem der Ansprüche 9 bis 11, dadurch gekennzeichnet, dass im zweiten Schritt das genannte Trägermaterial in einer Menge von 10 bis 400 Gew.-%, insbesondere von 20 bis 50 Gew.-%, bezogen auf die vorhandene Trockensubstanz, hinzugegeben wird, und dass die Hinzugabe vorzugsweise bei Raumtemperatur erfolgt.

13. Verfahren nach einem der Ansprüche 9 bis 12, dadurch gekennzeichnet, dass die Einengung im ersten und im dritten Schritt unter reduziertem Druck bei einer Temperatur von unterhalb 70°C erfolgt, wobei sich der jeweils angewendete Druck nach dem (den) verwendeten Lösungsmittel(n) richtet.

14. Verfahren nach einem der Ansprüche 9 bis 13, dadurch gekennzeichnet, dass die Restwassermenge in der eingeengten Masse weniger als 10 Gew.-%, vorzugsweise weniger als 5 Gew.-% beträgt, und dass die in der eingeengten Masse noch vorhandenen organischen Lösungsmittel dem gesetzlich maximal zugelassenen Wert entsprechen oder vorzugsweise diesen unterschreiten.

15. Verfahren nach einem der Ansprüche 9 bis 14, dadurch gekennzeichnet, dass am Ende des dritten Schrittes maximal 0,5 Gew.-% Ethanol, und/oder maximal 0,05 Gew.-% Methanol, und/oder maximal 0,05 Gew.-% 2-Propanol, und/oder maximal 20 ppm Methylethylketon, und/oder maximal 10 Gew.-%, insbesondere 5 Gew.-%, Wasser in der erhaltenen Masse vorhanden sind.

16. Verfahren nach einem der Ansprüche 9 bis 15, dadurch gekennzeichnet, dass man dem Gemisch zu einem beliebigen Zeitpunkt, vorzugsweise am Ende des ersten Schrittes, noch wenigstens einen Zusatz- und/oder Hilfsstoff hinzufügt, insbesondere ausgewählt aus der Gruppe, bestehend aus Emulgatoren, Stabilisatoren, Antioxidantien, Farbstoffen und Aromen.

## Patentansprüche (Patentansprüche für folgende(n) Vertragsstaat(en): ES)

1. Verfahren zur Herstellung einer Masse, welche insbesondere für die Einkapselung in ein festes Umhüllungsmaterial geeignet ist, dadurch gekennzeichnet, dass man
- in einem ersten Schritt einen auf übliche Art erhaltenen, wenigstens ein Extraktionsmittel enthaltenden Teil- oder Vollextrakt aus frischen und/oder getrockneten Pflanzen oder Teilen davon teilweise einengt,
- in einem zweiten Schritt den so erhaltenen Rückstand mit wenigstens einem Trägermaterial für den genannten Teil- oder Vollextrakt vermischt, wobei das genannte Trägermaterial verkapselbar und gegenüber dem genannten Extrakt inert sein muss, und
- in einem dritten Schritt das so erhaltene Gemisch nahezu vollständig vom Extraktionsmittel oder Extraktionsmittelgemisch befreit, und wobei
das Trägermaterial ausgewählt ist aus der Gruppe, bestehend aus
- Polyethylenglycolen der allgemeinen Formel
H-[-O-CH₂-CH₂-]ₙ-O-H
worin n eine ganze Zahl im Bereich von 100 bis 3000, insbesondere von 100 bis 400, bedeutet,
einschliesslich Gemische davon,
- Fettsäureestern von Polyglycerinen, einschliesslich Gemische davon,
- Siliconölen, einschliesslich Gemische davon,
- Lecithinen, einschliesslich Gemische davon,
- Sorbitanfettsäureestern, auch
- Sorbate von Fettsäuren genannt,
- Polysorbaten von Fettsäuren,
- Wachse,
- Polyglycerin,
- Triglyceride,
- Fettsäuren und
- fette Oele.

2. Verfahren nach Anspruch 1, dadurch gekennzeichnet, dass man am Ende des zweiten Schrittes einen gegebenenfalls entstandenen Niederschlag entfernt, beispielsweise mittels Filtration oder Zentrifugation.

3. Verfahren nach einem der Ansprüche 1 bis 2, dadurch gekennzeichnet, dass im ersten Schritt auf einen Trockensubstanzgehalt von etwa 20 bis 40 Gew.-% eingeengt wird.

4. Verfahren nach einem der Ansprüche 1 bis 3, dadurch gekennzeichnet, dass im zweiten Schritt das genannte Trägermaterial in einer Menge von 10 bis 400 Gew.-%, insbesondere von 20 bis 50 Gew.-%, bezogen auf die vorhandene Trockensubstanz, hinzugegeben wird, und dass die Hinzugabe vorzugsweise bei Raumtemperatur erfolgt.

5. Verfahren nach einem der Ansprüche 1 bis 4, dadurch gekennzeichnet, dass die Einengung im ersten und im dritten Schritt unter reduziertem Druck bei einer Temperatur von unterhalb 70°C erfolgt, wobei sich der jeweils angewendete Druck nach dem (den) verwendeten Lösungsmittel(n) richtet.

6. Verfahren nach einem der Ansprüche 1 bis 5, dadurch gekennzeichnet, dass die Restwassermenge in der eingeengten Masse weniger als 10 Gew.-%, vorzugsweise weniger als 5 Gew.-% beträgt, und dass die in der eingeengten Masse noch vorhandenen organischen Lösungsmittel dem gesetzlich maximal zugelassenen Wert entsprechen oder vorzugsweise diesen unterschreiten.

7. Verfahren nach einem der Ansprüche 1 bis 6, dadurch gekennzeichnet, dass am Ende des dritten Schrittes maximal 0,5 Gew.-% Ethanol, und/oder maximal 0,05 Gew.-% Methanol, und/oder maximal 0,05 Gew.-% 2-Propanol, und/oder maximal 20 ppm Methylethylketon, und/oder maximal 10 Gew.-%, insbesondere 5 Gew.-%, Wasser in der erhaltenen Masse vorhanden sind.

8. Verfahren nach einem der Ansprüche 1 bis 7, dadurch gekennzeichnet, dass man dem Gemisch zu einem beliebigen Zeitpunkt, vorzugsweise am Ende des ersten Schrittes, noch wenigstens einen Zusatz- und/oder Hilfsstoff hinzufügt, insbesondere ausgewählt aus der Gruppe, bestehend aus Emulgatoren, Stabilisatoren, Antioxidantien, Farbstoffen und Aromen.

9. Verfahren nach einem der Ansprüche 1 bis 8, dadurch gekennzeichnet, dass das Umhüllungsmaterial Gelatine ist, beispielsweise Weichgelatine oder Hartgelatine, wobei Weichgelatine bevorzugt ist.

10. Verfahren nach einen der Ansprüche 1 bis 9, dadurch gekennzeichnet, dass die Pflanzen Heil- oder Gewürzpflanzen sind, und insbesondere ausgewählt sind aus der Gruppe bestehend aus
Abelmoschus moschatus L. (Semen)
Acorus calamus (Rhizom)
Aesculus hippocastanum L. (Semen)
Allium-Arten (z.B. A. cepa L., A. ursinum L., A. sativum L.:Bulbus)
Alpinia officinarum Hance (Rhizom)
Arctostaphylos uva-ursi Spreng. (Folium)
Arnica montana L. (Flos)
Artemisia absinthium L. (Herba)
Artemisia dracunculus L. (Herba)
Atropa belladonna L. (Folium)
Berberis vulgaris L. (Cortex, Radix)
Betula-Arten (Folium)
Brassica nigra (L.) Koch (Semen)
Carum carvi L. (Fructus)
Cetraria islandica (L.) Ach.
Chrysanthemum vulgare Asch. (Herba)
Cinnamomum-Arten (Cortex)
Citrus-Arten (Folium, Flavedo, Fruct.)
Copaifera reticulata Ducke (Balsam)
Coriandrum sativum L. (Fructus)
Cucurbita pepo L. (Semen)
Cuninum cyminum L. (Fructus)
Curcuma-Varietäten (Rhizoma)
Cusparia officinalis (Willd.) Eng. (Cortex)
Dipterocarpus tubinatus Gaertn. (Balsamum)
Drosera-Arten (D. rotundifolia L., D.ramentacea Burch; Herba)
Echinacea angustifolia D.C. (Radix)
Echinacea purpurea (L.) Moench (Radix)
Elettaria cardamonum (L.) White et Mathon (Fructus)
Equisetum arvense L. (Herba)
Eucalyptus globulus Labill. (Folium)
Fagopyrum vulgare Hill. (Herba)
Foeniculum vulgare Miller (Fructus)
Gaultheria procumbens L. (Folium)
Ginkgo biloba L. (Folium)
Hamamelis virginiana L. (Cortex, Folium)
Hedeoma pulegioides (L.) Pers. (Herba)
Herniaria glabra L. (Herba)
Humulus lupulus L. (Flos, Glandulae)
Hypericum perforatum L. (Herba)
Hysopus officinalis L. (Herba)
Ilex paraguariensis St. Hil. (Folium mate)
Illicium verum Hook. f. (Fructus)
Iluna helenium L. (Rhizoma)
Iris pallida Lam. (Rhizoma)
Jasminum grandiflorum L. (Flos)
Laurus nobilis L. (Folium, Fructus)
Lavendula officinalis, weitere Arten (Flos)
Lawsonia inermis L. (Folium)
Levisticum officinale Koch (Radix)
Melaleuca: div. Arten (Folium)
Matricaria chamomilla L. (Flos)
Melilotus officinalis (L.) Lam. em. Thuill. (Herba)
Mentha-Arten und ihre Varietäten (Folium)
Myristica fragrans Houttuyn (Arillus, Semen)
Myrtus communis L. (Folium)
Ocimum basilicum L. (Herba)
Ocotea sassafras (Cortex)
Oenanthe aquatica (L.) Poir (Fructus)
Olibanum (Resinum)
Ononis spinosa L. (Radix)
Origanum-Varietäten (Herba)
Orthosiphon stamineus Benth. (Herba)
Panax ginseng Meyer (Radix)
Petroselinum crispum (Mill.) Nym. (Fructus, Herba)
Phaseolus vulgaris L. (Fructus sine Semine)
Pimenta dioica (L.) Merill (Fructus)
Pimpinella anisum L. (Semen)
Piper angustifolium Ruiz. et Pavon. (Folium)
Piper methysticum Forster (Radix)
Pogostemon patchouli Pell. (Folium)
Prunus laurocerasus L. (Folium)
Rhus aromatica Ait. (Cortex)
Rosmarinus officinalis L. und ihre Subspecies (Folium)
Rubia tinctorum L. (Radix)
Rubus fructicosus L. (Folium)
Ruta graveolens L. (Herba)
Sabal serulata Benth et Hook (Fructus)
Salix alba L. (Cortex) und alle Arten
Salvia-Varietäten (Folium)
Santalum album L. (Lignum)
Sarothamnus scoparius (L.) Wimmer (Herba)
Sassafras albidum (Nutt.) Nees (Lignum)
Satureja hortensis L. (Herba)
Scopolia carniolica Jacq. (Radix)
Solidago serotina Ait. (Herba)
Solidago virgaurea L. (Herba)
Syzygium aronaticum Merr. et Perry (Flores, Folium)
Taraxacum officinale Web. (Herba und Radix)
Thymus serpyllum L. (Herba)
Thymus vulgaris L. Herba
Tilia cordata Mill. und T.platyphyllos Scop. (Flos)
Urtica dioica L. (Folium, Radix)
Valeriana officinalis und ihre Varietäten (Radix)
Zingiberis officinale Roscoe (Rhizoma).

11. Verfahren nach einen der Ansprüche 1 bis 10, dadurch gekennzeichnet, dass das Gewichtsverhältnis von genanntem Extrakt zu genanntem Trägermaterial von 20 bis 200, insbesondere von 30 bis 150, vorzugsweise von 40 bis 150, beträgt.

12. Verfahren nach einem der Ansprüche 1 bis 11, dadurch gekennzeichnet, dass das Trägermaterial ein Polyethylenglycol ist, vorzugsweise Polyethylenglycol mit einem mittleren Polymerisationsgrad von 100 bis 3000, insbesondere 400.

## Claims (Claims for the following Contracting State(s): AT, BE, CH, LI, DE, DK, FR, GB, GR, IT, LU, MC, NL, PT, SE)

1. Mass, which is especially suitable for the encapsulation into a rigid covering material, characterized in that it contains
- at least one neither dried nor grinded partial extract or complete extract from fresh and/or dried plants or parts thereof, and
- at least one carrier material for said partial or complete extract,
whereby said carrier material must be encapsulatable and must be inert against said extract, and is selected from the group, consisting of
- polyethylenglycols of the general formula
H-[-O-CH₂-CH₂-]ₙ-O-H
wherein n is an integer in the range from 100 to 3000, especially from 100 to 400,
including mixtures thereof,
- fatty acid esters of polyglycerines, including mixtures thereof,
- silicone oils, including mixtures thereof,
- lecithins, including mixtures thereof,
- sorbitan fatty acid esters, also named sorbates of fatty acids,
- polysorbates of fatty acids,
- waxes,
- polyglycerin,
- triglycerides,
- fatty acids and
- fatty oils.

2. Mass according to claim 1, characterized in that it contains additionally water, especially in an amount up to 10 percent by weight, preferably in an amount up to 5 percent by weight.

3. Mass according to one of claims 1 to 2, characterized in that it contains additionally at least one organic solvent, especially in such an amount, which corresponds to the legally acceptable maximum value or which is preferably below this value, whereby maximum amounts of 0,5 percent by weight of ethanol, and/or maximum amounts of 0,05 percent by weight of methanol, and/or maximum amounts of 0,05 percent by weight of 2-propanol, and/or maximum amounts of 20 ppm of methylethylketon are preferred.

4. Mass according to one of claims 1 to 3, characterized in that the covering material is gelatine, for example soft gelatine or rigid gelatine, whereby soft gelatine is preferred.

5. Mass according to one of claims 1 to 4, characterized in that the plants are medical plants or spice plants, and are especially selected from the group consisting of
Abelmoschus moschatus L. (Semen)
Acorus calamus (Rhizom)
Aesculus hippocastanum L. (Semen)
Allium-species, (e.g. A. cepa L., A. ursinum L., A. sativum L.:Bulbus)
Alpinia officinarum Hance (Rhizom)
Arctostaphylos uva-ursi Spreng. (Folium)
Arnica montana L. (Flos)
Artemisia absinthium L. (Herba)
Artemisia dracunculus L. (Herba)
Atropa belladonna L. (Folium)
Berberis vulgaris L. (Cortex, Radix)
Betula-species (Folium)
Brassica nigra (L.) Koch (Semen)
Carum carvi L. (Fructus)
Cetraria islandica (L.) Ach.
Chrysanthemum vulgare Asch. (Herba)
Cinnamomum-species (Cortex)
Citrus-species (Folium, Flavedo, Fruct.)
Copaifera reticulata Ducke (Balsam)
Coriandrum sativum L. (Fructus)
Cucurbita pepo L. (Semen)
Cuminum cyminum L. (Fructus)
Curcuma-varieties (Rhizoma)
Cusparia officinalis (Willd.) Eng. (Cortex)
Dipterocarpus tubinatus Gaertn. (Balsamum)
Drosera species (D. rotundifolia L., D.ramentacea Burch; Herba)
Echinacea angustifolia D.C. (Radix)
Echinacea purpurea (L.) Moench (Radix)
Elettaria cardamonum (L.) White et Mathon (Fructus)
Equisetum arvense L. (Herba)
Eucalyptus globulus Labill. (Folium)
Fagopyrum vulgare Hill. (Herba)
Foeniculum vulgare Miller (Fructus)
Gaultheria procumbens L. (Folium)
Ginkgo biloba L. (Folium)
Hamamelis virginiana L. (Cortex, Folium)
Hedeoma pulegioides (L.) Pers. (Herba)
Herniaria glabra L. (Herba)
Humulus lupulus L. (Flos, Glandulae)
Hypericum perforatum L. (Herba)
Hysopus officinalis L. (Herba)
Ilex paraguariensis St. Hil. (Folium mate)
Illicium verum Hook. f. (Fructus)
Iluna helenium L. (Rhizoma)
Iris pallida Lam. (Rhizoma)
Jasminum grandiflorum L. (Flos)
Laurus nobilis L. (Folium, Fructus)
Lavendula officinalis, further species (Flos)
Lawsonia inermis L. (Folium)
Levisticum officinale Koch (Radix)
Maleleuca: various varieties (Folium)
Matricaria chamomilla L. (Flos)
Melilotus officinalis (L.) Lam. em. Thuill. (Herba)
Mentha-species and their varieties (Folium)
Myristica fragrans Houttuyn (Arillus, Semen)
Myrtus communis L. (Folium)
Ocimum basilicum L. (Herba)
Ocotea sassafras (Cortex)
Oenanthe aquatica (L.) Poir (Fructus)
Olibanum (Resinum)
Ononis spinosa L. (Radix)
Origanum-varieties (Herba)
Orthosiphon stamineus Benth. (Herba)
Panax ginseng Meyer (Radix)
Petroselinum crispum (Mill.) Nym. (Fructus, Herba)
Phaseolus vulgaris L. (Fructus sine Semine)
Pimenta dioica (L.) Merill (Fructus)
Pimpinella anisum L. (Semen)
Piper angustifolium Ruiz. et Pavon. (Folium)
Piper methysticum Forster (Radix)
Pogostemon patchouli Pell. (Folium)
Prunus laurocerasus L. (Folium)
Rhus aromatica Ait. (Cortex)
Rosmarinus officinalis L. and their subspecies (Folium)
Rubia tinctorum L. (Radix)
Rubus fructicosus L. (Folium)
Ruta graveolens L. (Herba)
Sabal serulata Benth et Hook (Fructus)
Salix alba L. (Cortex) and all species
Salvia-varieties (Folium)
Santalum album L. (Lignum)
Sarothamnus scoparius (L.) Wimmer (Herba)
Sassafras albidum (Nutt.) Nees (Lignum)
Satureja hortensis L. (Herba)
Scopolia carniolica Jacq. (Radix)
Solidago serotina Ait. (Herba)
Solidago virgaurea L. (Herba)
Syzygium aromaticum Merr. et Perry (Flores, Folium)
Taraxacum officinale Web. (Herba and Radix)
Thymus serpyllum L. (Herba)
Thymus vulgaris L. Herba
Tilia cordata Mill. and T.platyphyllos Scop. (Flos)
Urtica dioica L. (Folium, Radix)
Valeriana officinalis and their varieties (Radix)
Zingiberis officinale Roscoe (Rhizoma).

6. Mass according to one of claims 1 to 5, characterized in that the weight ratio of said extract to said carrier material is from 20 to 200, especially from 30 to 150, preferably from 40 to 150.

7. Mass according to one of claims 1 to 6, characterized in that the carrier material is a polyethylenglycol, preferably polyethylenglycol with an average polymerisation degree from 100 to 3000, especially 400.

8. Mass according to one of claims 1 to 7, characterized in that it contains additionally at least one additive and/or auxiliary agent, especially selected from the group, consisting of emulsifiers, stabilizers, antioxidants, dyestuffs and aromas.

9. A process for the preparation of a mass according to one of claims 1 to 8, characterized in that
- in a first step a conventionally obtained and at least one extraction agent containing partial or complete extract of fresh and/or dried plants or parts thereof is partially concentrated,
- in a second step the so obtained residue is mixed with at least one carrier material for said partial or complete extract, whereby said carrier material must be encapsulatable and must be inert against said extract, and
- in a third step the so obtained mixture is freed nearly completely from the extraction agent or the extraction agent mixture.

10. The process according to claim 9, characterized in that at the end of the second step an occasionally formed precipitate is removed, for example by means of filtration or centrifugation.

11. The process according to one of claims 9 to 10, characterized in that in the first step the mixture is concentrated to a solid matter content of from about 20 to 40 percent by weight.

12. The process according to one of claims 9 to 11, characterized in that in the second step said carrier material is added in an amount from 10 to 400 percent by weight, especially from 20 to 50 percent by weight, referred to the solid matter, which is present, and in that the addition is preferably realized at room temperature.

13. The process according to one of claims 9 to 12, characterized in that the concentrating in the first step and in the third step is realized under reduced pressure at a temperature of below 70°C, whereby in each case the applied pressure is regulated in dependency of the used solvent(s).

14. The process according to one of claims 9 to 13, characterized in that the residual water content in the concentrated mass is less than 10 percent by weight, preferably less than 5 percent by weight, and in that the organic solvents, which are still present in the concentrated mass, correspond to the legally acceptable maximum values or are preferably below these values.

15. The process according to one of claims 9 to 14, characterized in that at the end of the third step maximum amounts of 0,5 percent by weight of ethanol, and/or maximum amounts of 0,05 percent by weight of methanol, and/or maximum amounts of 0,05 percent by weight of 2-propanol, and/or maximum amounts of 20 ppm of methylethylketon, and/or maximum amounts of 10 percent by weight, especially 5 percent by weight, of water are contained in the obtained mass.

16. The process according to one of claims 9 to 15, characterized in that there is added to the mixture at any time, preferably at the end of the first step, additionally at least one additive and/or auxiliary agent, especially selected from the group, consisting of emulsifiers, stabilisators, antioxidants, dyestuffs and aromas.

## Claims (Claims for the following Contracting State(s): ES)

1. A process for the preparation of a mass which is especially suitable for the encapsulation into a rigid covering material, characterized in that
- in a first step a conventionally obtained and at least one extraction agent containing partial or complete extract of fresh and/or dried plants or parts thereof is partially concentrated,
- in a second step the so obtained residue is mixed with at least one carrier material for said partial or complete extract, whereby said carrier material must be encapsulatable and must be inert against said extract, and
- in a third step the so obtained mixture is freed nearly completely from the extraction agent or the extraction agent mixture, and whereby the carrier material is selected from the group, consisting of
- polyethylenglycols of the general formula
H-[-O-CH₂-CH₂-]ₙ-O-H
wherein n is an integer in the range from 100 to 3000, especially from 100 to 400,
including mixtures thereof,
- fatty acid esters of polyglycerines, including mixtures thereof,
- silicone oils, including mixtures thereof,
- lecithins, including mixtures thereof,
- sorbitan fatty acid esters, also named sorbates of fatty acids,
- polysorbates of fatty acids,
- waxes,
- polyglycerin,
- triglycerides,
- fatty acids and
- fatty oils.

2. The process according to claim 1, characterized in that at the end of the second step an occasionally formed precipitate is removed, for example by means of filtration or centrifugation.

3. The process according to one of claims 1 to 2, characterized in that in the first step the mixture is concentrated to a solid matter content of from about 20 to 40 percent by weight.

4. The process according to one of claims 1 to 3, characterized in that in the second step said carrier material is added in an amount from 10 to 400 percent by weight, especially from 20 to 50 percent by weight, referred to the solid matter, which is present, and in that the addition is preferably realized at room temperature.

5. The process according to one of claims 1 to 4, characterized in that the concentrating in the first step and in the third step is realized under reduced pressure at a temperature of below 70°C, whereby in each case the applied pressure is regulated in dependency of the used solvent(s).

6. The process according to one of claims 1 to 5, characterized in that the residual water content in the concentrated mass is less than 10 percent by weight, preferably less than 5 percent by weight, and in that the organic solvents, which are still present in the concentrated mass, correspond to the legally acceptable maximum values or are preferably below these values.

7. The process according to one of claims 1 to 6, characterized in that at the end of the third step maximum amounts of 0,5 percent by weight of ethanol, and/or maximum amounts of 0,05 percent by weight of methanol, and/or maximum amounts of 0,05 percent by weight of 2-propanol, and/or maximum amounts of 20 ppm of methylethylketon, and/or maximum amounts of 10 percent by weight, especially 5 percent by weight, of water are contained in the obtained mass.

8. The process according to one of claims 1 to 7, characterized in that there is added to the mixture at any time, preferably at the end of the first step, additionally at least one additive and/or auxiliary agent, especially selected from the group, consisting of emulsifiers, stabilisators, antioxidants, dyestuffs and aromas.

9. The process according to one of claims 1 to 8, characterized in that the covering material is gelatine, for example soft gelatine or rigid gelatine, whereby soft gelatine is preferred.

10. The process according to one of claims 1 to 9, characterized in that the plants are medical plants or spice plants, and are especially selected from the group consisting of
Abelmoschus moschatus L. (Semen)
Acorus calamus (Rhizom)
Aesculus hippocastanum L. (Semen)
Allium-species, (e.g. A. cepa L., A. ursinum L., A. sativum L.:Bulbus)
Alpinia officinarum Hance (Rhizom)
Arctostaphylos uva-ursi Spreng. (Folium)
Arnica montana L. (Flos)
Artemisia absinthium L. (Herba)
Artemisia dracunculus L. (Herba)
Atropa belladonna L. (Folium)
Berberis vulgaris L. (Cortex, Radix)
Betula-species (Folium)
Brassica nigra (L.) Koch (Semen)
Carum carvi L. (Fructus)
Cetraria islandica (L.) Ach.
Chrysanthemum vulgare Asch. (Herba)
Cinnamomum-species (Cortex)
Citrus-species (Folium, Flavedo, Fruct.)
Copaifera reticulata Ducke (Balsam)
Coriandrum sativum L. (Fructus)
Cucurbita pepo L. (Semen)
Cuminum cyminum L. (Fructus)
Curcuma-varieties (Rhizoma)
Cusparia officinalis (Willd.) Eng. (Cortex)
Dipterocarpus tubinatus Gaertn. (Balsamum)
Drosera species (D. rotundifolia L., D.ramentacea Burch; Herba)
Echinacea angustifolia D.C. (Radix)
Echinacea purpurea (L.) Moench (Radix)
Elettaria cardamonum (L.) White et Mathon (Fructus)
Equisetum arvense L. (Herba)
Eucalyptus globulus Labill. (Folium)
Fagopyrum vulgare Hill. (Herba)
Foeniculum vulgare Miller (Fructus)
Gaultheria procumbens L. (Folium)
Ginkgo biloba L. (Folium)
Hamamelis virginiana L. (Cortex, Folium)
Hedeoma pulegioides (L.) Pers. (Herba)
Herniaria glabra L. (Herba)
Humulus lupulus L. (Flos, Glandulae)
Hypericum perforatum L. (Herba)
Hysopus officinalis L. (Herba)
Ilex paraguariensis St. Hil. (Folium mate)
Illicium verum Hook. f. (Fructus)
Iluna helenium L. (Rhizoma)
Iris pallida Lam. (Rhizoma)
Jasminum grandiflorum L. (Flos)
Laurus nobilis L. (Folium, Fructus)
Lavendula officinalis, further species (Flos)
Lawsonia inermis L. (Folium)
Levisticum officinale Koch (Radix)
Melaleuca: various varieties (Folium)
Matricaria chamomilla L. (Flos)
Melilotus officinalis (L.) Lam. em. Thuill. (Herba)
Mentha-species and their varieties (Folium)
Myristica fragrans Houttuyn (Arillus, Semen)
Myrtus communis L. (Folium)
Ocimum basilicum L. (Herba)
Ocotea sassafras (Cortex)
Oenanthe aquatica (L.) Poir (Fructus)
Olibanum (Resinum)
Ononis spinosa L. (Radix)
Origanum-varieties (Herba)
Orthosiphon stamineus Benth. (Herba)
Panax ginseng Meyer (Radix)
Petroselinum crispum (Mill.) Nym. (Fructus, Herba)
Phaseolus vulgaris L. (Fructus sine Semine)
Pimenta dioica (L.) Merill (Fructus)
Pimpinella anisum L. (Semen)
Piper angustifolium Ruiz. et Pavon. (Folium)
Piper methysticum Forster (Radix)
Pogostemon patchouli Pell. (Folium)
Prunus laurocerasus L. (Folium)
Rhus aromatica Ait. (Cortex)
Rosmarinus officinalis L. and their subspecies (Folium)
Rubia tinctorum L. (Radix)
Rubus fructicosus L. (Folium)
Ruta graveolens L. (Herba)
Sabal serulata Benth et Hook (Fructus)
Salix alba L. (Cortex) and all species
Salvia-varieties (Folium)
Santalum album L. (Lignum)
Sarothamnus scoparius (L.) Wimmer (Herba)
Sassafras albidum (Nutt.) Nees (Lignum)
Satureja hortensis L. (Herba)
Scopolia carniolica Jacq. (Radix)
Solidago serotina Ait. (Herba)
Solidago virgaurea L. (Herba)
Syzygium aromaticum Merr. et Perry (Flores, Folium)
Taraxacum officinale Web. (Herba and Radix)
Thymus serpyllum L. (Herba)
Thymus vulgaris L. Herba
Tilia cordata Mill. and T.platyphyllos Scop. (Flos)
Urtica dioica L. (Folium, Radix)
Valeriana officinalis and their varieties (Radix)
Zingiberis officinale Roscoe (Rhizoma).

11. The process according to one of claims 1 to 10, characterized in that the weight ratio of said extract to said carrier material is from 20 to 200, especially from 30 to 150, preferably from 40 to 150.

12. The process according to one of claims 1 to 11, characterized in that the carrier material is a polyethylenglycol, preferably polyethylenglycol with an average polymerisation degree from 100 to 3000, especially 400.

## Revendications (Revendications pour l'(les) Etat(s) contractant(s) suivant(s): AT, BE, CH, LI, DE, DK, FR, GB, GR, IT, LU, MC, NL, PT, SE)

1. Masse appropriée en particulier pour l'encapsulation dans un matériau d'enrobage solide,
caractérisée en ce qu'elle contient:
- au moins un extrait partiel ou total non séché ni broyé de plantes ou de parties de plantes fraîches et/ou séchées, et
- au moins un matériau de support pour ledit extrait partiel ou total,
dans laquelle le matériau support précité doit être encapsulable et être inerte vis-à-vis de l'extrait précité et est choisi dans le groupe constitué de
- les polyéthylèneglycols de formule générale
H-[-O-CH₂-CH₂-]ₙ-O-H
dans laquelle n représente un nombre entier compris dans l'intervalle de 100 à 3000, et en particulier de 100 à 400,
y compris leurs mélanges,
- les esters d'acides gras des polyglycérines, y compris leurs mélanges,
- les huiles de silicone, y compris leurs mélanges,
- les lécithines, y compris leurs mélanges,
- les esters d'acides gras de sorbitol, également appelés les sorbates d'acides gras,
- les polysorbates d'acides gras,
- les cires,
- la polyglycérine,
- les triglycérides,
- les acides gras, et
- les huiles grasses.

2. Masse selon la revendication 1, caractérisée en ce qu'elle contient encore de l'eau, en particulier en quantité jusqu'à 10% en poids et, de préférence, en quantité jusqu'à 5% en poids;

3. Masse selon l'une des revendications 1 à 2, caractérisée en ce qu'elle contient encore au moins un solvant organique et, en particulier, en quantité telle que celle-ci corresponde à la valeur maximale autorisée légalement et soit, de préférence, inférieure à cette valeur, dans laquelle des quantités de 0,5% en poids au maximum d'éthanol et/ou de 0,05% en poids au maximum de méthanol et/ou 0,05% en poids au maximum de 2-propanol et/ou 20 ppm au maximum de méthyléthylcétone sont préférées.

4. Masse selon l'une des revendications 1 à 3, caractérisée en ce que le matériau d'enrobage est de la gélatine et, par exemple, de la gélatine molle ou de la gélatine dure, la gélatine molle étant préférée.

5. Masse selon l'une des revendications 1 à 4, caractérisée en ce que les plantes sont des plantes médicinales et/ou aromatiques et sont choisies, en particulier, dans le groupe constitué de:
Abelmoschus moschatus L. (semence)
Acorus calamus (rhizome)
Aesculus hippocastanum L. (semence)
Allium (espèces) (p. ex.A. cepa L., A. ursinum L., A. sativum L.: bulbe)
Alpinia officinarum Hance (rhizome)
Arctostaphylos uva-ursi Spreng. (feuilles)
Arnica montana L. (fleurs)
Artemisia absinthium L. (herbe)
Artemisia dracunculus L. (herbe)
Atropa belladona L. (feuilles)
Berberis vulgaris L. (écorce, racine)
Betula (espèces) (feuilles)
Brassica nigra (L.) Koch (semence)
Carum carvi L. (fruits)
Cetraria islandica (L.) Ach.
Chrysanthemum vulgare Asch. (herbe)
Cinnamomum (espèces) (écorce)
Citrus (espèces) (feuilles, flavedo, fruits)
Copaifera reticulata Ducke (baume)
Coriandrum sativum L. (fruits)
Cucurbita pepo L. (semence)
Cuminum cyminum L. (fruits)
Curcuma (variétés de) (rhizome)
Cuspari officinalis (Willd.) Eng. (écorce)
Dipterocarpus tubinatus Gaertn. (balsamum)
Drosera (variétés) (D. rotundifolia L., D. ramentacea Burch; Herbe)
Echinacea angustifolia D.C. (racine)
Echinacea purpurea (L.) Moench (racine)
Elettaria cardamonum (L.) White et Mathon (fruits)
Equisetum arvense L. (herbe)
Eucalyptus globulus Labill. (feuilles)
Fagopyrum valgare Hill. (herbe)
Foeniculum vulgare Miller (fruits)
Gaultheria procumbens L. (feuilles)
Ginkgo biloba L. (feuilles)
Hamamelis virginiana L. (écorce, feuilles)
Hedeoma pulegioides (L.) Pers. (herbe)
Herniaria glabra L. (herbe)
Humulus lupulus L. (fleurs, graines)
Hypericum perforatum L. (herbe)
Hysopus officinalis L. (herbe)
Ilex paraguariensis St. Hil. (feuilles)
Illicium verum Hook. f. (fruits)
Iluna helenium L. (rhizome)
Iris pallida Lam. (rhizome)
Jasminum grandiflorum L. (fleurs)
Laurus nobilis L. (feuilles, fruits)
Lavendula officinalis (espèces de) (fleurs)
Lawsonia inermis L. (feuilles)
Levisticum officinale Koch (racine)
Maleleuca: espèces diverses (feuilles)
Matricaria chamomilla L. (fleurs)
Melilotus officinalis (L.) Lam. em. Thuill. (herbe)
Mentha (espèces et leurs variétés) (feuilles)
Myristica fragrans Houttuyn (arillus, semen)
Myrtus communis L. (feuilles)
Ocimum basilicum L. (herbe)
Ocotea sassafras (écorce)
Oenanthe aquatica (L.) Poir (fruits)
Olibanum (résine)
Ononis spinosa L. (racine)
Origanum (variétés de) (herbe)
Orthosiphon stamineus Benth. (herbe)
Panax ginseng Meyer (racine)
Petroselinum crispum (Mill.) Nym. (fruits, herbe)
Phaseolus vulgaris L. (fruits sans semence)
Pimenta dioica (L.) Merill (fruits)
Pimpinella anisum L. (semence)
Piper angustifolium Ruiz. et Pavon. (feuilles)
Piper methysticum Forster (racine)
Pogostemon patchouli Pell. (feuilles)
Prunus laurocerasus L. (feuilles)
Rhus aromatica Ait. (écorce)
Rosmarinus officinalis L. et ses sous-espèces (feuilles)
Rubia tinctorum L. (racine)
Rubus fructicosus L. (feuilles)
Ruta graveolens L. (herbe)
Sabal serulata Benth et Hook (fruits)
Salix alba L. (écorce) et toutes variétés
Salvia (variétés de) (feuilles)
Santalum album L. (bois)
Sarothamnus scoparius (L.) Wimmer (herbe)
Sassafras albidum (Nutt.) Nees (bois)
Satureja hortensis L. (herbe)
Scopolia carniolica Jacq. (racine)
Solidago serotina Ait. (herbe)
Solidago virgaurea L. (herbe)
Syzygium aromaticum Merr. et Perry (fleurs, feuilles)
Taraxacum officinale Web. (herbe et racine)
Thymus serpyllum L. (herbe)
Thymus vulgaris L. (herbe)
Tilia cordata Mill. et T. platyphyllos Scop. (fleurs)
Urtica dioica L. (feuilles, racine)
Valeriana officinalis et ses variétés (racine)
Zingiberis officinale Roscoe (rhizome).

6. Masse selon l'une des revendications 1 à 5, caractérisée en ce que le rapport pondéral de l'extrait précité au matériau support précité est compris entre 20 et 200 et, en particulier, entre 30 et 150 et, de préférence, entre 40 et 150.

7. Masse selon l'une des revendications 1 à 6, caractérisée en ce que le matériau support est un polyéthylèneglycol et, de préférence, un polyéthylèneglycol ayant un degré moyen de polymérisation entre 100 et 3000 et égal plus particulièrement à 400.

8. Masse selon l'une des revendications 1 à 7, caractérisée en ce qu'elle contient encore au moins un additif et/ou un adjuvant, choisi en particulier dans le groupe constitué des émulsifiants, des stabilisants, des antioxydants, des colorants et des aromates.

9. Procédé de préparation d'une masse selon l'une des revendications 1 à 8, caractérisé en ce que:
- au cours d'une première étape, on concentre partiellement un extrait partiel ou total, obtenu de la manière usuelle et contenant au moins un agent d'extraction provenant de plantes fraîches et/ou séchées,
- au cours d'une deuxième étape, on mélange le résidu ainsi obtenu avec au moins un matériau de support pour l'extrait partiel ou total précité, ledit matériau de support devant être encapsulable et inerte vis à vis de l'extrait précité, et
- au cours d'une troisième étape, on élimine presque complètement du mélange ainsi obtenu l'agent d'extraction ou le mélange d'agent d'extraction.

10. Procédé selon la revendication 9, caractérisé en ce qu'on élimine un précipité produit éventuellement à la fin de la deuxième étape, par exemple par filtration ou centrifugation.

11. Procédé selon l'une des revendications 9 et 10, caractérisé en ce qu'on concentre, au cours de la première étape, pour obtenir une teneur en substances sèches comprise entre environ 20 et 40% en poids.

12. Procédé selon l'une des revendications 9 à 11, caractérisé en ce que, au cours de la deuxième étape, on ajoute le matériau support précité en quantité comprise entre 10 et 400% en poids et, en particulier, entre 20 et 50% en poids, rapportée à la substance sèche existante et en ce que l'addition s'effectue de préférence à température ambiante.

13. Procédé selon l'une des revendications 9 à 12, caractérisé en ce que la concentration au cours de la première étape et de la troisième étape est effectuée sous pression réduite, à une température inférieure à 70°C, la pression utilisée dans chaque cas dépendant du ou des solvant(s) utilisé(s).

14. Procédé selon l'une des revendications 9 à 13, caractérisé en ce que la quantité d'eau résiduelle dans la masse concentrée est inférieure à 10% en poids et est, de préférence, inférieure à 5% en poids et ce que la masse concentrée contient encore des solvants organiques présents en quantité correspondant à la valeur maximale autorisée légalement ou étant, de préférence, inférieure à cette valeur.

15. Procédé selon l'une des revendications 9 à 14, caractérisé en ce que, à la fin de la troisième étape, il reste dans la masse obtenue au maximum 0,5% en poids d'éthanol et/ou au maximum 0,05% en poids de méthanol et/ou au maximum 0,05% en poids de 2-propanol et/ou au maximum 20 ppm de méthyléthylcétone et/ou au maximum 10% en poids et, plus particulièrement, 5% en poids d'eau.

16. Procédé selon l'une des revendications 9 à 15, caractérisé en ce que l'on ajoute au mélange, à un moment quelconque et, de préférence à la fin de la première étape, encore au moins un additif et/ou un adjuvant choisi en particulier dans le groupe constitué des émulsifiants, des stabilisants, des antioxydants, des colorants et des aromates.

## Revendications (Revendications pour l'(les) Etat(s) contractant(s) suivant(s): ES)

1. Procédé de préparation d'une masse qui est appropriée en particulier pour l'encapsulation dans un matériau d'enrobage solide, caractérisé en ce que:
- au cours d'une première étape, on concentre partiellement un extrait partiel ou total, obtenu de la manière usuelle et contenant au moins un agent d'extraction provenant de plantes fraîches et/ou séchées,
- au cours d'une deuxième étape, on mélange le résidu ainsi obtenu avec au moins un matériau de support pour l'extrait partiel ou total précité, ledit matériau de support devant être encapsulable et inerte vis à vis de l'extrait précité, et
- au cours d'une troisième étape, on élimine presque complètement l'agent d'extraction ou le mélange d'agent d'extraction du mélange ainsi obtenu, et dans lequel le matériau de support est choisi dans le groupe constitué de:
- les polyéthylèneglycols de formule générale
H-[-O-CH₂-CH₂-]ₙ-O-H
dans laquelle n représente un nombre entier compris dans l'intervalle de 100 à 3000, et en particulier de 100 à 400,
y compris leurs mélanges,
- les esters d'acides gras des polyglycérines, y compris leurs mélanges,
- les huiles de silicone, y compris leurs mélanges,
- les lécithines, y compris leurs mélanges,
- les esters d'acides gras de sorbitol également appelés les sorbates d'acides gras,
- les polysorbates d'acides gras,
- les cires,
- la polyglycérine,
- les triglycérides,
- les acides gras, et
- les huiles grasses.

2. Procédé selon la revendication 1, caractérisé en ce, à la fin de la deuxième étape, on élimine un précipité produit éventuellement, par exemple par filtration ou centrifugation.

3. Procédé selon l'une des revendications 1 et 2, caractérisé en ce que, au cours de la première étape, on concentre pour obtenir une teneur en substances sèches comprise entre environ 20 et 40% en poids.

4. Procédé selon l'une des revendications 1 à 3, caractérisé en ce qu'au cours de la deuxième étape, on ajoute le matériau support précité en quantité comprise entre 10 et 400% en poids et, en particulier, entre 20 et 50% en poids, rapportée à la substance sèche existante et en ce que l'addition s'effectue de préférence à température ambiante.

5. Procédé selon l'une des revendications 1 à 4, caractérisé en ce que la concentration au cours de la première étape et de la troisième étape est effectuée sous pression réduite, à une température inférieure à 70°C, la pression utilisée dans chaque cas dépendant du ou des solvant(s) utilisé(s).

6. Procédé selon l'une des revendications 1 à 5, caractérisé en ce que la quantité d'eau résiduelle dans la masse concentrée est inférieure à 10% en poids et est, de préférence, inférieure à 5% en poids et ce que la masse concentrée contient encore des solvants organiques présents en quantité correspondant à la valeur maximale autorisée légalement ou étant, de préférence, inférieure à cette valeur.

7. Procédé selon l'une des revendications 1 à 6, caractérisé en ce que, à la fin de la troisième étape, il reste dans la masse obtenue au maximum 0,5% en poids d'éthanol et/ou au maximum 0,05% en poids de méthanol et/ou au maximum 0,05% en poids de 2-propanol et/ou au maximum 20 ppm de méthyléthylcétone et/ou au maximum 10% en poids et, plus particulièrement, 5% en poids d'eau.

8. Procédé selon l'une des revendications 1 à 7, caractérisé en ce que l'on ajoute au mélange, à un moment quelconque et, de préférence à la fin de la première étape, encore au moins un additif et/ou un adjuvant choisi en particulier dans le groupe constitué des émulsifiants, des stabilisants, des antioxydants, des colorants et des aromates.

9. Procédé selon l'une des revendications 1 à 8, caractérisé en ce que le matériau d'enrobage est de la gélatine et, par exemple, de la gélatine molle ou de la gélatine dure, la gélatine molle étant préférée.

10. Procédé selon l'une des revendications 1 à 9, caractérisé en ce que les plantes sont des plantes médicinales ou aromatiques et sont choisies, en particulier dans le groupe constitué de:
Abelmoschus moschatus L. (semence)
Acorus calamus (rhizome)
Aesculus hippocastanum L. (semence)
Allium (espèces) (p. ex.A. cepa L., A. ursinum L., A. sativum L.: bulbe)
Alpinia officinarum Hance (rhizome)
Arctostaphylos uva-ursi Spreng. (feuilles)
Arnica montana L. (fleurs)
Artemisia absinthium L. (herbe)
Artemisia dracunculus L. (herbe)
Atropa belladona L. (feuilles)
Berberis vulgaris L. (écorce, racine)
Betula (espèces) (feuilles)
Brassica nigra (L.) Koch (semence)
Carum carvi L. (fruits)
Cetraria islandica (L.) Ach.
Chrysanthemum vulgare Asch. (herbe)
Cinnamomum (espèces) (écorce)
Citrus (espèces) (feuilles, flavedo, fruits)
Copaifera reticulata Ducke (baume)
Coriandrum sativum L. (fruits)
Cucurbita pepo L. (semence)
Cuminum cyminum L. (fruits)
Curcuma (variétés de) (rhizome)
Cuspari officinalis (Willd.) Eng. (écorce)
Dipterocarpus tubinatus Gaertn. (balsamum)
Drosera (variétés) (D. rotundifolia L., D. ramentacea Burch; Herbe)
Echinacea angustifolia D.C. (racine)
Echinacea purpurea (L.) Moench (racine)
Elettaria cardamonum (L.) White et Mathon (fruits)
Equisetum arvense L. (herbe)
Eucalyptus globulus Labill. (feuilles)
Fagopyrum valgare Hill. (herbe)
Foeniculum vulgare Miller (fruits)
Gaultheria procumbens L. (feuilles)
Ginkgo biloba L. (feuilles)
Hamamelis virginiana L. (écorce, feuilles)
Hedeoma pulegioides (L.) Pers. (herbe)
Herniaria glabra L. (herbe)
Humulus lupulus L. (fleurs, graines)
Hypericum perforatum L. (herbe)
Hysopus officinalis L. (herbe)
Ilex paraguariensis St. Hil. (feuilles)
Illicium verum Hook. f. (fruits)
Iluna helenium L. (rhizome)
Iris pallida Lam. (rhizome)
Jasminum grandiflorum L. (fleurs)
Laurus nobilis L. (feuilles, fruits)
Lavendula officinalis (espèces de) (fleurs)
Lawsonia inermis L. (feuilles)
Levisticum officinale Koch (racine)
Melaleuca: espèces diverses (feuilles)
Matricaria chamomilla L. (fleurs)
Melilotus officinalis (L.) Lam. em. Thuill. (herbe)
Mentha (espèces et leurs variétés) (feuilles)
Myristica fragrans Houttuyn (arillus, semen)
Myrtus communis L. (feuilles)
Ocimum basilicum L. (herbe)
Ocotea sassafras (écorce)
Oenanthe aquatica (L.) Poir (fruits)
Olibanum (résine)
Ononis spinosa L. (racine)
Origanum (variétés de) (herbe)
Orthosiphon stamineus Benth. (herbe)
Panax ginseng Meyer (racine)
Petroselinum crispum (Mill.) Nym. (fruits, herbe)
Phaseolus vulgaris L. (fruits sans semence)
Pimenta dioica (L.) Merill (fruits)
Pimpinella anisum L. (semence)
Piper angustifolium Ruiz. et Pavon. (feuilles)
Piper methysticum Forster (racine)
Pogostemon patchouli Pell. (feuilles)
Prunus laurocerasus L. (feuilles)
Rhus aromatica Ait. (écorce)
Rosmarinus officinalis L. et ses sous-espèces (feuilles)
Rubia tinctorum L. (racine)
Rubus fructicosus L. (feuilles)
Ruta graveolens L. (herbe)
Sabal serulata Benth et Hook (fruits)
Salix alba L. (écorce) et toutes variétés
Salvia (variétés de) (feuilles)
Santalum album L. (bois)
Sarothamnus scoparius (L.) Wimmer (herbe)
Sassafras albidum (Nutt.) Nees (bois)
Satureja hortensis L. (herbe)
Scopolia carniolica Jacq. (racine)
Solidago serotina Ait. (herbe)
Solidago virgaurea L. (herbe)
Syzygium aromaticum Merr. et Perry (fleurs, feuilles)
Taraxacum officinale Web. (herbe et racine)
Thymus serpyllum L. (herbe)
Thymus vulgaris L. (herbe)
Tilia cordata Mill. et T. platyphyllos Scop. (fleurs)
Urtica dioica L. (feuilles, racine)
Valeriana officinalis et ses variétés (racine)
Zingiberis officinale Roscoe (rhizome).

11. Procédé selon l'une des revendications 1 à 10, caractérisé en ce que le rapport pondéral de l'extrait précité au matériau support précité est compris entre 20 et 200 et, en particulier, entre 30 et 150 et, de préférence, entre 40 et 150.

12. Procédé selon l'une des revendications 1 à 11, caractérisé en ce que le matériau support est un polyéthylèneglycol et, de préférence, un polyéthylèneglycol ayant un degré moyen de polymérisation entre 100 et 3000 et égal plus particulièrement à 400.
